# NEW EUROPEAN PATENT SPECIFICATION

(11) **EP 1 224 215 B2**
(45) Date of publication and mention of the opposition decision: **06.10.2010**
(45) Mention of the grant of the patent: 17.08.2005
(21) Application number: 00975093.6
(22) Date of filing: 24.10.2000
(51) Int. Cl.: C07K 14/415, A61K 39/36, A61P 37/08

(54) **NON-ANAPHYLACTIC FORMS OF GRASS POLLEN Ph1 p 6 ALLERGEN AND THEIR USE**
NICHT-ANAPHYLAKTISCHE FORMEN DES GRASSPOLLENALLERGENS PH1 P 6 UND DEREN VERWENDUNG
FORMES ANAPHYLACTIQUE D'ALLERGENE DE POLLEN DE GRAMINEES Phl p 6 ET LEUR UTILISATION

(30) Priority: 29.10.1999 SE 9903950
(43) Date of publication of application: 24.07.2002
(73) Proprietor: Phadia AB, 751 37 Uppsala (SE); Valenta, Rudolf, 2604 Theresienfeld (AT)
(72) Inventor: VALENTA, Rudolf, A-2604 Theresienfeld (AT); VRTALA, Susanne, A-1210 Vienna (AT); STUMMVOLL, Sabine, A-3352 St. Peter (AT); GRÖNLUND, Hans, S-181 57 Lidingö (SE); GROTE, Monika, D-48161 Münster (DE); VANGELISTA, Luca, IT- 35122 Padova (IT); PASTORE, Annalisa, c/o Molecular Structure Div, London NW7 1AA (GB); SPERR, Wolfgang, R., A-1190 Vienna (AT); VALENT, Peter, A-1170 Vienna (AT); KRAFT, Dietrich, A-1170 Vienna (AT)
(74) Representative: Lindgren, Anders
(86) International application number: PCT/SE2000/002062
(87) International publication number: WO 2001/030816

(56) References cited:
- WO-A2-98/43657
- SUSANNE VRTALA ET AL.: 'Molecular, immunological and structural characterization of Ph1 p 6, a major allergen and P-particle-associated protein from Timothy grass (Phleum pratense) pollen' J. IMMUNOL. vol. 163, no. 10, November 1999, pages 5489 - 5496, XP002938145
- A. PETERSEN ET AL.: 'Characterization of the allergen group VI in Timothy grass pollen (Ph1 p6) II. cDNA cloning of Ph1 6 and structural comparison to grass group V' INT. ARCH. ALLERGY IMMUNOL. vol. 108, 1995, pages 55 - 59, XP002938146
- GABRIELE SCHRAMM ET AL.: 'Allergen engineering: Variants of the Timothy grass pollen allergen Ph1 p 5b with reduced IgE-binding capacity but conserved T cell reactivity' J. IMMUNOL. vol. 162, no. 4, February 1999, pages 2406 - 2414, XP002938147

## Description

The present invention relates to non-anaphylactic, i.e. hypoallergenic, forms of the major timothy grass pollen allergen Phl p 6 for hyposensitization and for diagnosis.

Type I allergy is a genetically determined hypersensitivity disease that affects more than 20% of the population in industrialized countries (1). As a consequence of this immuno-disorder, allergic patients produce IgE antibodies against *per se* innocuous, mostly air-born proteins from pollen, mites, moulds and animal hair/dander. The symptoms of Type I allergy (allergic rhinitis, conjunctivitis, allergic asthma and anaphylactic shock) result from allergen-induced crosslinking of effector cell (mast cell, basophil)-bound IgE antibodies and subsequent release of inflammatory mediators (2). Since approximately 40% of allergic individuals suffer symptoms following contact with grass pollen, research has concentrated on the characterization of relevant grass pollen allergens by protein and immunochemical methods (3). While groups of major allergens have been identified as cross-reactive moieties that occur in most grass species (4), nothing was known concerning their nature and biological functions.

The recent application of molecular biological techniques to allergen characterization has revealed the primary structures of allergens and facilitated the production of recombinant allergens for diagnostic and therapeutic purposes (5). Components of the plant cytoskeleton (e. g., profilin) (6) as well as calcium-binding pollen proteins (7) have been identified as relevant allergens. The fact that allergic patients exhibit immediate type reactions upon contact with various unrelated allergen sources thus can be explained by cross-reactivity of their IgE antibodies with ubiquitous allergens. Evidence that group 1 grass pollen allergens belong to a family of cell wall-loosening proteins (expansins) (8) and grass group 5 allergens may possess RNAse activity (9) has restimulated ideas that the biological function of a given protein may be related to its allergenicity. The recent findings that major grass pollen allergens can either become attached to small sized particles (e. g., group 1 allergens to diesel exhaust (10)) or may become airborn as small pollen subcompartments (e. g., group 5 allergens in amyloplasts (11)) would provide a possible mechanism of how certain allergens may be able to reach the deep airways of patients and to elicit allergic asthma.

Therapy of Type I allergic diseases is currently performed by pharmacological treatment and by specific immunotherapy. Specific immunotherapy has been established already early in this century (Noon, Lancet 1 (1911) 1572-1573) and involves the systemic application of increasing doses of allergens for extended periods. Although specific immunotherapy is recognized as effective treatment, the occurrence of anaphylactic side effects represents one of the major disadvantages of this therapy. To reduce anaphylactic reactions the use of T-cell epitopes has recently been proposed for allergen specific immunotherapy (Briner et al., Proc. Natl. Acad. Sci. USA 90 (1993) 7608-7612, and Norman, Curr. Opin. Immunol. 5 (1993) 986-973). Allergens harbour a great variety of different T-cell epitopes (Ebner et al., J. Immunol 150 (1993) 1047-1054; Joost-van-Neerven et al., J. Immunol. 151 (1993) 2326-2335; and Schenket al., J. Allergy Clin. Immunol. 96 (1995) 986-996) which may overlap with continuous IgE-epitopes. To prevent crosslinking of effector cell (mast cell, basophil) bound IgE and mediator release, T-cell epitopes and IgE epitopes need to be dissected.

Vrtala et al., J. Clin. Invest. 99(7) 1673-1681 (1997) and WO 99/16467 disclose a novel strategy of reducing the anaphylactic activity of the major birch allergen Bet v 1 by disrupting the three dimensional structure by expressing two parts of the Bet v 1 cDNA representing amino acids 1-74 and 75-160 in *Escherichia coli.* In contrast to the complete recombinant Bet v 1, the recombinant fragments showed almost no allergenicity. Both non-anaphylactic fragments induced proliferation of human Bet v 1-specific T cell clones, indicating that they harboured all dominant T cell epitopes and therefore could be used for safe and specific T cell immunotherapy. The success of this strategy was believed to be due to the fact that the Bet v 1 allergen possesses discontinuous (i.e. conformational) IgE epitopes rather than continuous IgE epitopes as is the case for many other allergens.

In contrast to the major birch allergen Bet v 1, the major timothy grass pollen allergen Phl p 6 contains continuous (sequential) IgE epitopes and would therefore not be susceptible to the above fragmentation strategy to reduce anaphylactic activity as outlined above.

According to the present invention, however, it has surprisingly and most unexpectedly been found that Phl p 6 deletion variants may be constructed by genetic (recombinant) or synthetic fragmentation, which fragments may be used for specific immunotherapy of grass pollen allergy with reduced anaphylactic side effects. Such fragments with strongly reduced anaphylactic ability will below be referred to as non-anaphylactic or hypoallergenic.

The N-terminal or C-terminal deletion mentioned below may be a terminal truncation of the allergen- The deletion may also be internal within the N-terminal or C-terminal part of the allergen, respectively.

The allergen molecule fragments may be produced by recombinant DNA techniques or peptide synthetic chemistry as is *per se* well known to the skilled person.

In one aspect, the present invention provides a hypoallergenic immunogenic combination of molecules derived from the Phl p 6 allergen, comprising (i) a Phl p 6 molecule having an N-terminal deletion which makes the molecule at least substantially lack IgE binding capacity, and (ii) a Phl p 6 molecule having a C-terminal deletion which makes the molecule at least substantially lack IgE binding capacity, which two molecules together encompass the complete amino acid sequence of the Phl p 6 allergen.

For together encompassing the complete amino acid sequence of Phl p 6, the respective sequences of the two Phl p 6 molecules may overlap or be contiguous.

The sizes of the N-terminal and C-terminal deletions of the Phl p 6 allergen necessary for the fragments to be useful for the purposes of the invention, i.e. that the fragments are (i) immunogenic and (ii) non-IgE reactive, may readily be determined by the skilled person. Thus, the lack or presence of IgE binding ability of a particular N-terminal or C-terminally deletion molecule may easily be determined, the lack of IgE reactivity indicating that the molecule may be applied without or with low risk of inducing anaphylactic side effects. Immunogenic activity of the molecules may be determined by their capability of being recognized by a polyclonal antiserum to the complete Phl p 6 allergen. In this way fragments and fragment combinations, respectively, may be selected which have a very high likelyhood of being capable of eliciting immune responses which protect against the complete allergen.

A further aspect of the invention is the use of the hypoallergenic immunogenic combination of molecules in a specific hyposensitization therapy. Such therapy may be performed as known in the art for protein allergens and encompasses administering repeatedly to the mammal, typically a human individual, suffering from type I allergy against the allergen an immunogen that is capable of raising an IgG immune response against the allergen. The immunogen may be admixed with suitable adjuvants such as aluminium oxide. Administration may be done systemically, for instance by injection, infusion, etc, but also the oral route has been suggested in order to expose the intestinal part of the immune system. See also Norman PS, "Current status of immunotherapy for allergies and anaphylactic reactions" Adv. Internal. Medicine 41 (1996)681-713.

Here, the immunogen to be administered is the above-mentioned hypoallergenic immunogenic combination of molecules derived from the Phl p 6 allergen, i.e. (i) a Phl p 6 molecule having an N-terminal deletion which makes the molecule at least substantially lack IgE binding capacity, and (ii) a Phl p 6 molecule having a C-terminal deletion which makes the molecule at least substantially lack IgE binding capacity, which two molecules together encompass the complete amino acid sequence of Phl p 6.

A further aspect of the present invention provides the use of the combination of immunogens as an antigen in an immunoassay for detecting specific antibodies of the IgA, IgD, IgE, IgG or IgM class directed against the Phl p 6 allergen from which the immunogen(s) derive. Appropriate assay variants involve formation of a ternary immune complex between the immunogen, sample antibody and an antibody directed against the Ig-class of interest. The sample may be any Ig-containing biological fluids, for instance a blood derived sample (serum, plasma, whole blood), CSF, etc. Especially, the hypoallergenic fragments may be used for diagnostic monitoring (e.g. IgG measurements, measurement of T cell responses) during therapy when inducing a new immune response against the fragments. The invention will now be illustrated by the following non-limiting Example.

### EXAMPLE

### MATERIALS AND METHODS

### Biological materials, patients sera, antisera, recombinant allergens.

Pollen from timothy grass (*Phleum pratense*), rye grass (*Lolium perenne*)*,* rye *(Secale cereale),* Kentucky blue grass (*Poa pratensis*)*,* wheat (*Triticum sativum*)*,* cultivated oat (*Avena sativa*) and common reed (*Phragmites communis*) were from Allergon AB, (Välinge, Sweden). Timothy grass seeds were purchased from Austrosaat, (Vienna, Austria) and grown for 4 weeks to obtain fresh leaves and roots. Patients allergic to grass pollen were characterized as described (4). The rabbit anti-celery profilin antiserum (RP1) is described (12). A rabbit anti-rPhl p 6 antiserum was raised against purified, recombinant Phl p 6 using Freunds adjuvans (Charles River, Kissleg, Germany). Recombinant timothy grass pollen allergens, rPhl p 1, rPhl p 2 and rPhl p 5 were purified as described (13). Recombinant timothy grass pollen profilin was purified by poly (L-proline) affinity chromatography (6).

### Isolation and characterization of cDNAs coding for Phl p 6 isoforms/fragments.

Threehundred and fifty IgE-reactive clones were isolated from an expression cDNA library constructed from mature timothy grass pollen in phage λgt 11 (14). Six cDNAs (c121, c142, c146, c171, c223, c233) with sequence homology to a Phl p 6-encoding cDNA (15) were subcloned into plasmid pUC18 and sequenced (16, 17). Sequences were analyzed using the McVector program (Kodak, Rochester, NY). Search for Phl p 6-homologous protein sequences was done with the FastA program (GCG package) (18) in the SwissProt database. The sequences of Hol 15 and Hor v 5 allergens were retrieved from the EMBL database. Multiple sequence alignment was produced with ClustalW (19) and edited by hand. The GDE sequence editor (S. Smith, Harvard University, Cambridge, MA) and COLORMASK (J. Thompson, EMBL, Heidelberg, Germany) were used to colour conserved residues with related properties (19). Protein secondary structure and surface accessibility predictions were done with the PHD program on the EMBL PredictProtein server (20).

### Mapping of Phl p 6 IgE epitopes, expression and purification of recombinant Phl p 6.

The IgE binding capacity of phage clones expressing Phl p 6 isoforms and fragments was investigated by a plaque lift assay (21). The DNA coding for the mature Phl p 6 allergen was PCR-amplified from the clone 142 DNA, subcloned into the NdeI/Eco R I site of pET-17b. Recombinant Phl p 6 was expressed in *E*. *coli* BL 21 (DE 3) in liquid culture. Cells were suspended in 25 mM Imidazole, pH 7.4, 0.1% Triton X-100 and lysed by addition of lysozyme (20 µg/g cells) for 30 minutes at room temperature as well as by freeze-thawing cycles. DNA was digested with DNAse I (0.1 mg/g cell pellet) for 20 minutes at room temperature. The protein extract was centrifuged for 20 min at 10.000xg (Sorvall RC5C; SS34 rotor) to remove insoluble materials. rPhl p 6 was enriched in a precipitate obtained by addition of ammonium sulfate (40-60% w/v). The precipitate was dissolved in 10mM Tris pH 6, dialyzed against this buffer and after centrifugation (20min, 10.000g, Sorvall RC5C; SS34 rotor) was applied to a diethylaminoethyl cellulose-Sepharose column (Pharmacia). Unbound proteins were eluted with 10 mM Tris, pH 6, 4% v/v isopropanol. Fractions containing more than 80% pure Phl p 6 were adjusted to pH 8 with NaOH and subjected to a second chromatography step on a diethylaminoethyl cellulose-Sepharose column. Elution of bound proteins with a 0-0.5 M NaCI gradient at pH 8 yielded fractions containing pure rPhl p 6 which were dialyzed against H₂O_{dd}.

### MALDI-TOF (Matrix assisted laser desorption and ionisation - time of flight) and CD (circular dichroism) analysis of purifted recombinant Phl p 6.

Laser desorption mass spectra were acquired in a linear mode with a time-of-flight Compact MALDI II instrument (Kratos, Manchester, UK) (piCHEM, Graz, Austria). CD spectra were recorded on a Jasco J-710 spectropolarimeter fitted with a Jasco PTC-348WI peltier type temperature control system and interfaced with a Fisons HAAKE GH water bath. Far ultraviolet CD spectra were recorded at 20°C in a 2 mm path-length quartz cuvette (Hellma, Mullheim, Baden, Germany) at a protein concentration of 7 µM. Thermal denaturation of Phl p 6 was monitored by recording the ellipticity during temperature increase (50°C/h) at 220 nm. The reversibility of the unfolding process was checked by measuring the restoration of the CD signal upon cooling (50°C/h) to the starting temperature (20°C). The fraction of folded protein was calculated as F=1-U, where U=(Θ₂₂₀-Θ_{N})/(Θ_{U}-Θ_{N}). Θ_{N} is the ellipticity of the protein in the native state and Θ_{U} that of the denatured protein. For rPhl p 6, Θ_{U} was assumed to be equal to Θ₂₂₀ at 85°C and Θ_{N} to Θ₂₂₀ at 20°C.

### IgE-binding capacity of recombinant Phl p 6, cross-reactivity with natural Phl p 6 and other timothy grass pollen allergens.

The prevalence of IgE anti-rPhl p 6 reactivity was determined in sera from 171 grass pollen allergic patients and, for control purposes, in sera from non-atopic persons by ELISA (13). The presence of cross-reactive IgE epitopes on natural and rPhl p 6 was investigated by IgE immunoblot inhibition experiments (4). A possible immunological relationship between rPhl p 6 and recombinant timothy grass pollen allergens (rPhl p 1, rPhl p 2, rPhl p 5) (13) was studied by ELISA competition as described (4).

### Histamine release experiments.

Granulocytes were isolated from heparinized blood samples of grass pollen allergic individuals containing rPhl p 6-reactive IgE antibodies by dextran sedimentation (22). Cells were incubated with increasing concentrations of purified rPhl p 5, rPhl p 6, and with an anti-human IgE antibody (E 124.2.8 Dε2, Immunotech, Marseille, France). Histamine released into the supematants was measured by radioimmunoassay (Immunotech, Marseille, France).

### Skin testing.

After informed consent was obtained skin prick tests were performed on the forearms of the individuals as described (23). Individuals were pricked with 20 µl aliquots containing different concentrations (1 µg/ml, 10 µg/ml, 100 µg/ml) of purified rPhl p 6, rPhl p 5 and with timothy grass pollen extract, histamine and sodium chloride (ALK, Horsholm, Denmark).

### Analysis of the presence of Phl p 6-related allergens in other grass species and tissue-specific expression of Phl p 6.

Protein extracts from pollens, leaves and roots were obtained by homogenizing the tissues in SDS-sample buffer (24). Insoluble materials were removed by centrifuging the extracts (10.000xg, 20 min; Sorvall RC5C, SS34 rotor). Protein extracts were separated by 14% SDS-PAGE (25) and blotted onto nitrocellulose (26). Nitrocellulose strips were probed with a rabbit anti-celery profilin antiserum, RP1, (12), the rabbit anti-rPhl p 6 antiserum and the latter rabbits preimmune serum. Bound rabbit antibodies were detected with a 1:1000 diluted ¹²⁵I-labeled donkey anti-rabbit Ig antiserum (Amersham).

### In situ localization of Phl p 6 by immunogold electron microscopy.

Timothy grass pollen grains were unhydrously fixed as described (27). Ultrathin sections were incubated with equal concentrations of either rabbit anti-rPhl p 6 Ig (Ig: protein G-purified immunoglobulin fraction) or preimmune Ig. Bound rabbit antibodies were detected with goat anti-rabbit IgG antibodies coupled to 10 nm colloidal gold particles (Plano, Wetzlar, Germany) (27).

### Construction of hypoallergenic Phl p 6 (Phleum pratense) deletion variants.

N-terminal and C-terminal Phl p 6 deletion variants were generated to represent aa 1-57 and aa 31-110. cDNAs coding for Phl p 6 aa 1-57 and Phl p 6 aa 31-110 were obtained by PCR amplification of the Phl p 6 cDNA (clone #142) using the following oligonucleotide primers:
For Phl p 6 aa 1-57:
   5': GG*G AAT TC*C ATA TGG GGA AGG CCA CGA CC 3' 
5': CGG GGT ACC CTA GTG GTG GTG GTG GTG GTG GGG CGC CTT TGA
AAC 3'
For Phl p 6 aa 31-110:
   5': GG*G AAT TC*C ATA TGG CAG ACA AGT ATA AG 34 
5’:.CCG GAA TTC CTA GTG GTG GTG GTG GTG GTG CGC GCC GGG CTT
GAC 3’
Eco R I and Kpn I sites are printed in italics, Nde I sites and a His-tag, which has been introduced at the C-terminus, are underlined.
The PCR-products were cut with Nde I/Kpn I (aa 1-57) or with Nde I/Eco R I (aa 31-110), purified by preparative agarose gel electrophoresis, subcloned into plasmid pET-17b (Novagen) and transformed into *E. coli* BL 21(DE3) (Novagen). Colonies expressing the correct deletion variants were identified by immunoscreening using a rabbit anti-Phl p 6 antiserum. DNA from positive clones was isolated using Qiagen tips (Qiagen, Hilden, Germany) and sequenced (MWG-Biotech, Hilden, Germany).

### Expression of Phl p 6 deletion variants in E. coli and testing of their IgE-binding capacity

Recombinant Phl p 6 aa 1-57 and Phl p 6 aa 31-110 were expressed in *E. coli* B1 21 (DE 3) by induction with 0.5 mM isopropyl-β-thiogalactopyranoside at an OD₆₀₀ of 0.8 in liquid culture for 5 h at 37°C. Equal amounts of rPhl p 6, rPhl p 6 aa 1-57 and rPhl p 6 aa 31-110 were separated by SDS-PAGE and blotted onto nitrocellulose.
Nitrocellulose strips were incubated with serum IgE from allergic individuals, nonallergic control persons, with a rabbit anti-Phl p 6 antiserum and a rabbit preimmunserum. Bound IgE antibodies were detected with ¹²⁵I-labeled anti-human IgE antibodies and bound rabbit antibodies with ¹²⁵I-labeled donkey anti-rabbit antibodies.

### RESULTS

### Isolation and characterization of cDNAs coding for isoforms/fragments of Phl p 6.

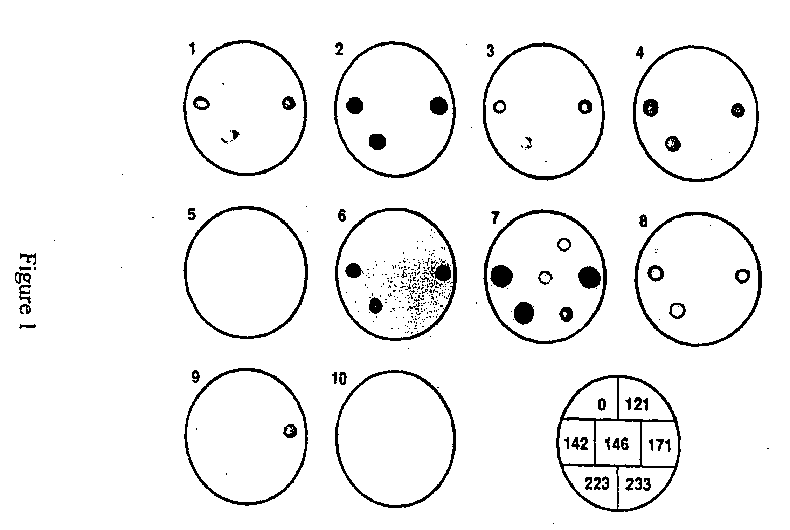

Six cDNA clones (c142, c223, c171, c121, c233, c146), coding for Phl p 6 isoforms/fragments were isolated from a timothy grass pollen λgt11 library with serum IgE from a grass pollen allergic patient. The sequences of the described clones have been deposited in the GenBank database (Accession numbers: Y16955-Y16960). The deduced amino acid sequence of Phl p 6 (clone 142) contained a 28 aa hydrophobic leader peptide. A molecular mass of 11.8 kDa and a pl of 5.5 were calculated for the mature Phl p 6 (clone 142) protein which starts with a glycine residue and shows a high content of alanine residues (20.9%). The computer-aided secondary structure analysis of Phl p 6 indicates a predominant helical content and the calculation of solvent accessibility predicts that many of the N-terminal amino acids are solvent exposed while most of the C-terminal amino acids appeared buried. A search for sequence motifs revealed the presence of one potential N-linked glycosylation site (NAS: aa 15-17), one N-terminal myristoylation site (GKAT: aa 1-4), two cAMP-dependent protein kinase phosphorylation sites (KATT: aa 2-5; KYKT: aa 33-36) and two peroxisomal targeting sequences (GKA: aa 1-3; SKA: aa 54-56). The deduced Phl p 6 amino acid sequence displayed identity with a recently submitted Phl p 6 sequence (15) and similarities with the N-terminal portions of group 5 grass pollen allergens. However, Phl p 6 specific IgE shows little or no crossreactivity with group 5 allergens. A comparison with group 5 grass pollen allergens is given in Vrtala, S., et al., J. Immunol. 1999, 163(10): 5489-5496 (37) (the disclosure of which is incorporated by reference herein). Figure 1A therein shows a multiple sequence alignment, secondary structure and solvent accessibility prediction of Phl p 6 variants and group 5 allergens.

### The Phl p 6 N-terminus is relevant for IgE binding.

Nitrocellulose-bound β-gal-fused complete (c223, c142), N-terminally truncated rPhl p 6 (c171, c121, c233, c146) and, for control purposes, β-gal alone were exposed to serum IgE from 9 grass pollen allergic individuals and a non-allergic person (Figure 1). Results obtained showed that the two complete Phl p 6 isoforms and a Phl p 6 fragment lacking only 4 of the N-terminal amino acids strongly bound IgE from all grass pollen allergic patients tested and that the IgE binding capacity of the partial Phl p 6 clones decreased depending on the number of amino acids which were absent from the proteins' N-terminus. A partial clone (clone 121) lacking the N-terminal 30 amino acids had almost completely lost its IgE binding capacity (Figure 1).

### E. coli expression and purification of recombinant Phl p 6. IgE binding capacity of purified rPhl p 6.

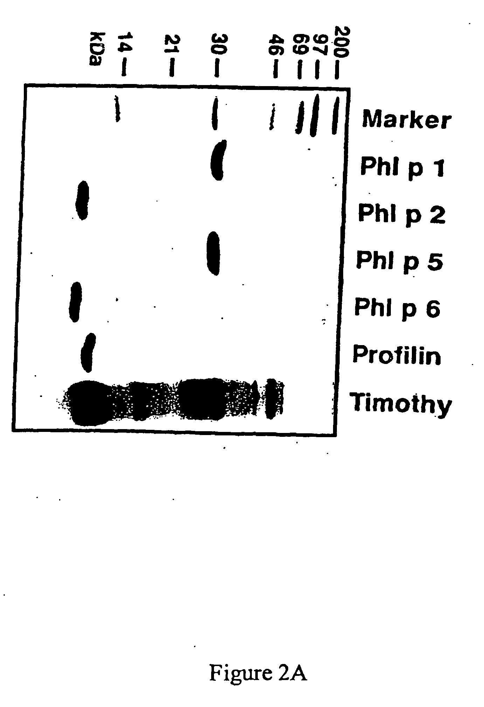

rPhl p 6 was overexpressed in *E. coli* BL21 (DE3). A combination of several purification steps yielded pure and soluble rPhl p 6 (approximately 5mg protein/liter *E. coli* culture) which by SDS-PAGE was identified as one of the low molecular weight timothy grass pollen allergens (Figure 2A). MALDI-TOF analysis of purified recombinant Phl p 6 resulted in two mass/charge peaks of 11790 and 5896 corresponding to the MH+ and M2H2+ species of the sample which were in agreement with the deduced Phl p 6 molecular mass (11789 Da).

In 128 sera from 171 grass pollen allergic patients but in no serum from 10 non-allergic individuals rPhl p 6-specific IgE antibodies were detected. Preabsorption of sera from grass pollen allergic patients with rPhl p 6 led to a great reduction of IgE binding to a 10-14 kDa moiety in nitrocellulose-blotted timothy grass pollen extract indicating that rPhl p 6 and natural Phl p 6 share IgE epitopes. ELISA competition experiments demonstrated that only a small percentage (<20%) of Phl p 5-specific IgE could be preabsorbed with rPhl p 6. IgE binding to rPhl p 1, rPhl p 2 and recombinant timothy grass profilin was not reduced after preincubation of sera with rPhl p 6. These results identify Phl p 6 as a major allergen which is distinct from other grass pollen allergens.

### rPhl p 6 folds in a stable all alpha helical conformation.

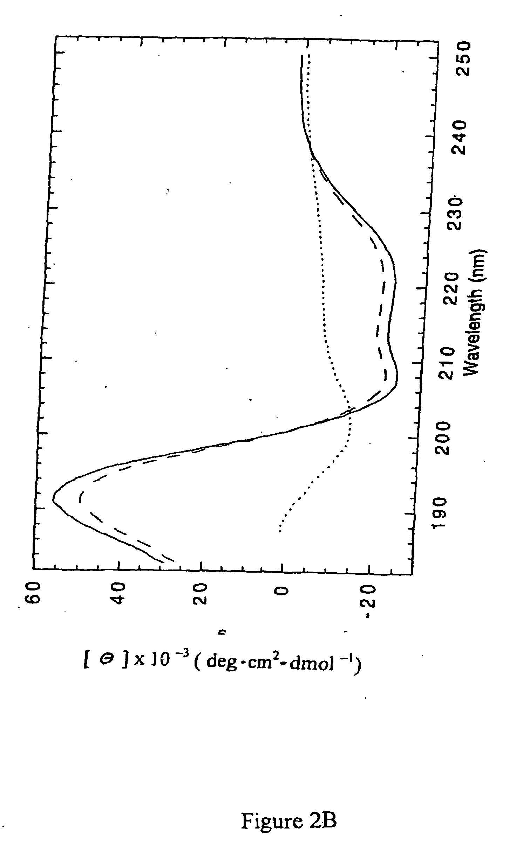

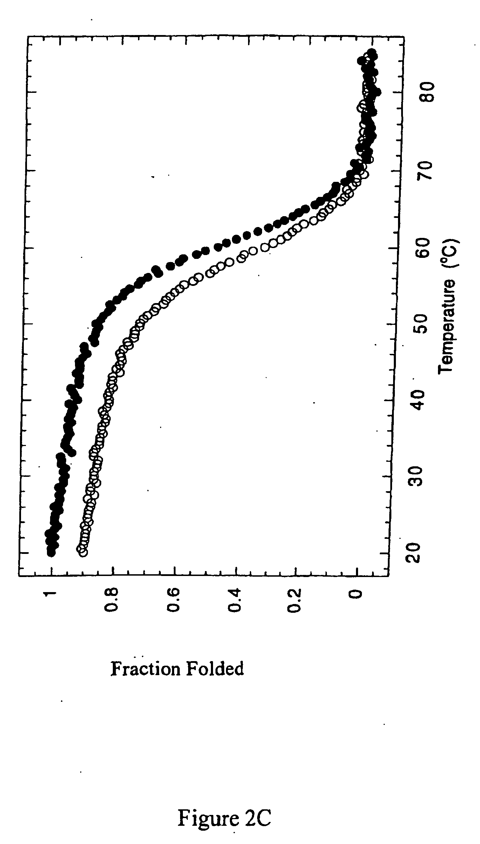

The far-ultraviolet CD spectrum of purified rPhl p 6 (Figure 2B) indicates that the protein contains a considerable amount of alpha-helical secondary structure. The spectrum is characterized by two broad minima at 208nm and 220nm and a maximum at 191nm. The secondary structure prediction (37) is in good agreement with the CD measurements as it indicates predominant alpha helical secondary structure content. The unfolding transition of rPhl p 6 is monophasic and highly cooperative with a melting point of 61 °C. At 85°C, rPhl p 6 assumes a random coil conformation, with a typical minimum at 200nm. rPhl p 6 shows a high degree of folding reversibility, evident from the cooling curve profile (Figure 2C) and the far-UV spectrum recorded at 20°C after cooling from 85°C (Figure 2B).

### Recombinant Phl p 6 induces dose dependent basophil histamine release and immediate type skin reactions in grass pollen allergic patients.

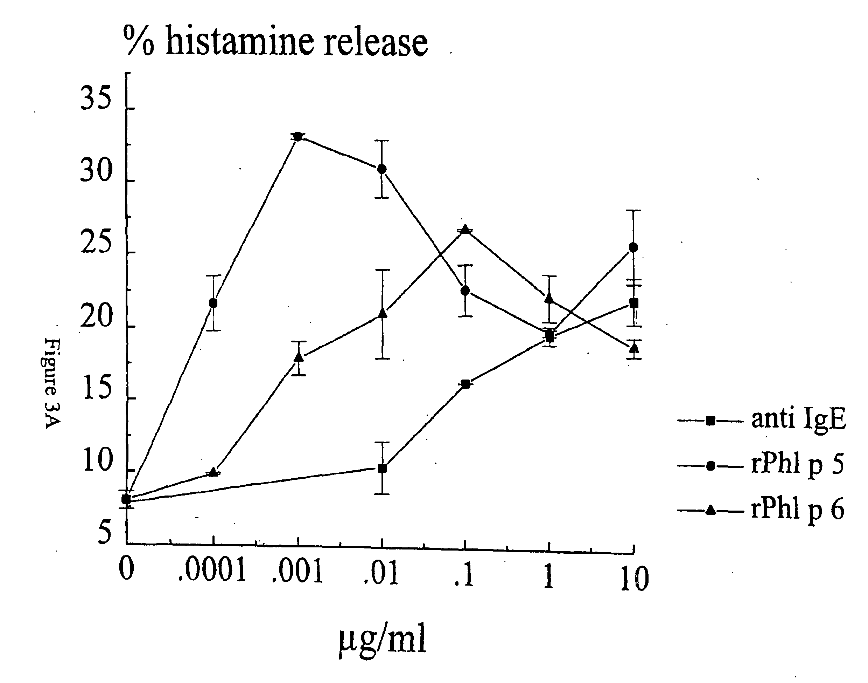

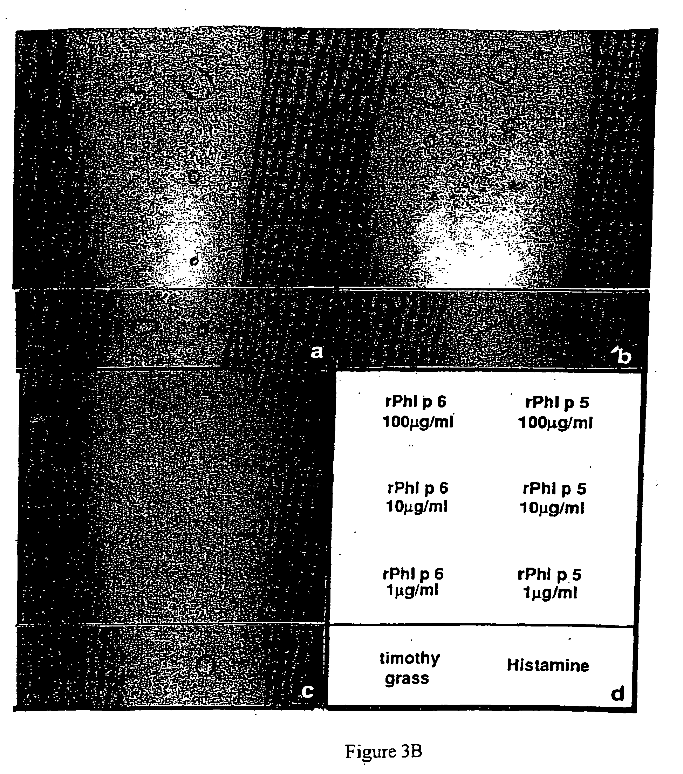

Purified rPhl p 6 induced specific and dose-dependent histamine release from basophils of a grass pollen allergic patient (Figure 3A). rPhl p 5 which represents a highly active grass pollen allergen (14, Valenta and Flicker, unpublished data) induced maximal release already at a lower concentration compared to rPhl p 6. In four grass pollen allergic patients but not in the non-allergic individuals, rPhl p 6, rPhl p 5 and timothy grass pollen extract induced immediate type skin reactions (Table 1; Figure 3B). While no reactions to sodium chloride were observed, histamine induced wheal reactions in all individuals tested (Table 1; Figure 3B).

### Group 6 allergens represent pollen-specific proteins.

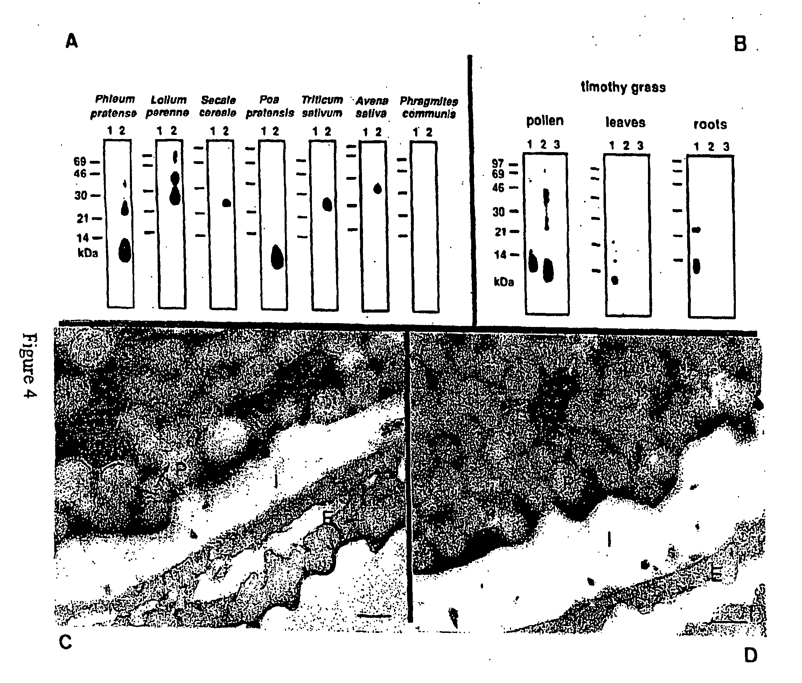

While major groups of grass pollen allergens occur in pollens of most grass species (4), group 6 allergens were reported to occur exclusively in timothy grass *(Phleum pratense)* pollen (15). A rabbit anti-rPhl p 6 antiserum cross-reacted with group 5 allergens in nitrocellulose blotted pollen extracts from various monocots (*Phleum pratense, Lolium perenne, Secale cereale, Triticum sativum, Avena sativa, Phragmites communis*) between 25-28 kDa (Figure 4A, lanes 2). Phl p 6 or Phl p 6-related allergens at 11 kDa were detected exclusively in pollens from *Phleum pratense* and *Poa pratensis.* Although a putative N-glycosylation site was found in the aminoacid sequence deduced from the Phl p 6 cDNA sequence, comparable molecular weights observed for natural and recombinant Phl p 6 exclude heavy glycosylation of natural Phl p 6 (Figures 4A, 2A). Rabbit anti-rPhl p 6 antibodies strongly reacted with Phl p 6 at 11 kDa in nitrocellulose-blotted timothy grass pollen but not with leaf or root extracts (Figure 4B, lanes 2). Profilin was detected in all three tissues at approximately 14 kDa (Figure 4B, lanes 1).

### Immunelectronmicroscopical localization of Phl p 6 in timothy grass pollen.

Using post-embedding immunogold electron microscopy, rabbit anti-rPhl p 6 antibodies bound to the numerous polysaccharide (P-) particles which fill much of the interior of a mature timothy grass pollen grain (Figure 4C). The greatest accumulation of gold particles was observed on sectioned surfaces of the P-particles indicating that Phl p 6 is present on rather than in the P-particles. Little (cytosol, exine) or no (mitochondria, intine) anti-rPhl p 6 immunoreactivity was observed in other parts of the pollen grain. Likewise almost no gold particles were detected in the amyloplasts. This localization pattern, taken together with our finding that a rabbit anti-rPhl p 5 antiserum failed to label the P-particles (data not shown) excludes the possibility that the immunolabeling of the P-particles resulted from the presence of cross-reactive group 5 allergens. Control experiments performed with preimmune Ig yielded only a few non-specifically adsorbed gold particles (Figure 4D).

### Phl p 6 deletion variants (aa 1-57, aa 31-110) exhibit strongly reduced IgE binding capacity.

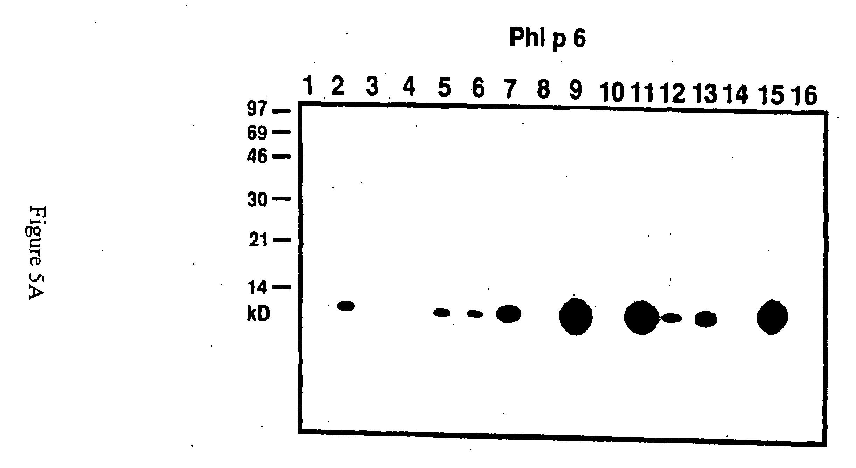

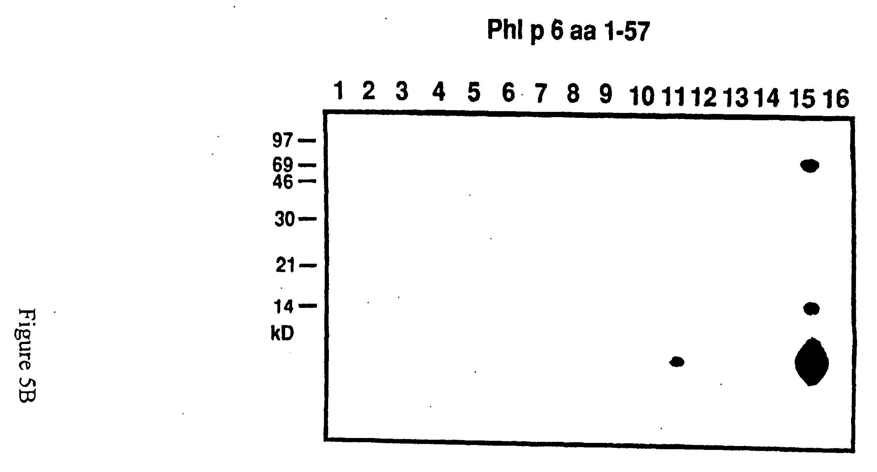

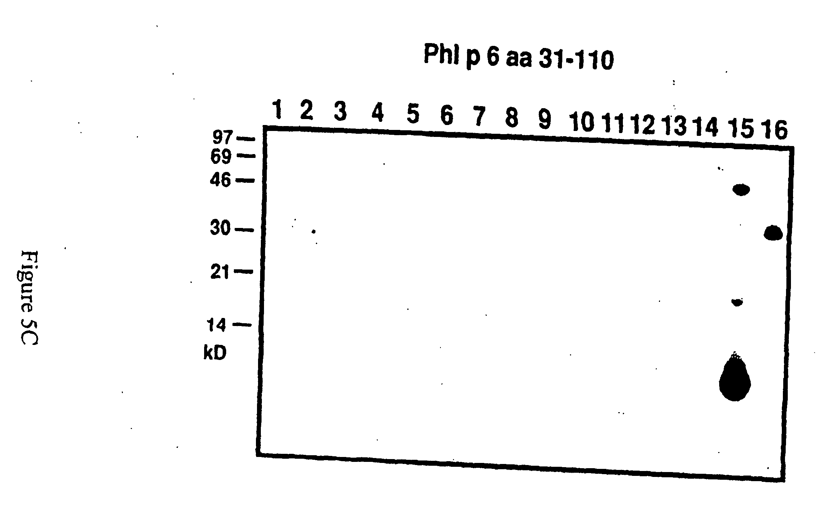

Nitrocellulose-blotted complete rPhl p 6 (Figure 5A), rPhl p 6 variant aa 1-57 (Figure 5B) and rPhl p 6 variant aa 31-110 (Figure 5C) were exposed to 13 sera from grass pollen allergic patients, to a serum from a non-atopic person and to a rabbit anti-rPhl p 6 antiserum. While all 13 grass pollen allergic patients displayed IgE reactivity to complete recombinant Phl p 6 (Figure 5A), variant aa 1-57 was recognized by serum 11 and weakly by serum 13 (Figure 5B). Phl p 6 variant aa 31-110 reacted only weakly with serum 7 and 11 (Figure 5C). Serum from the non-atopic individual failed to react with complete rPhl p 6 and the deletion variants. The rabbit anti-rPhl p 6 antiserum showed reactivity of comparable intensity to complete rPhl p 6 and the two deletion variants (Figures 5A-C: lanes 15) whereas the rabbits preimmune serum showed no reactivity in the molecular weight range of the molecules (Figures 5A-C: lanes 16).

### IgG1-reactivity of mouse anti-rPhl p 6 or anti-rPhl p 6 aa 31-110 antisera to rPhl p 6

Mouse IgG1 raised against complete rPhl p 6 and rPhl p 6 aa 31-110 react with rPhl p 6 (Table II).

### rPhl p 6 derivatives have a greatly reduced capacity to induce histamine release

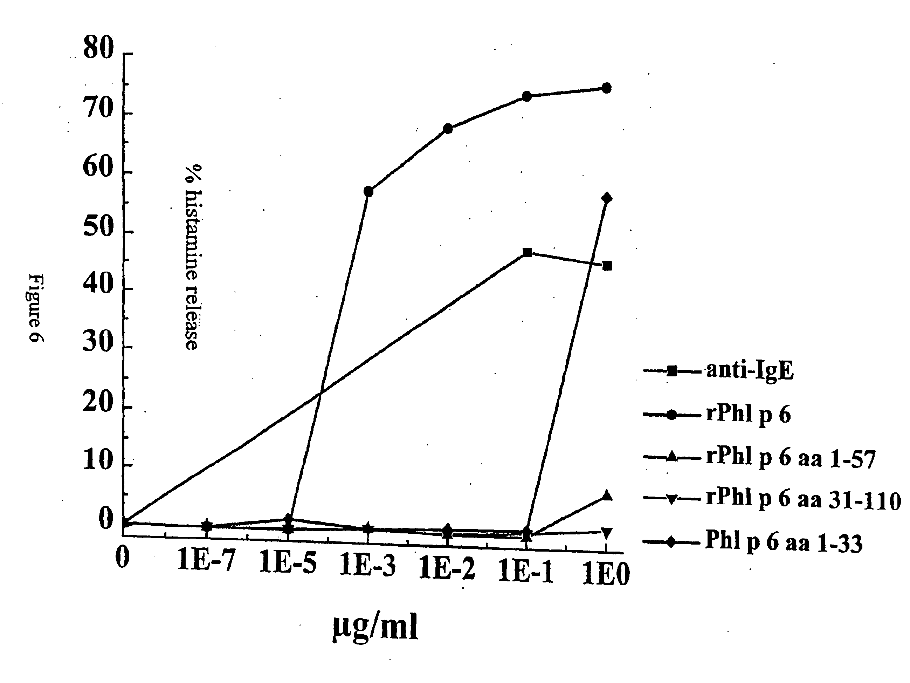

Granulocytes from a patient allergic to grass pollen were incubated with various concentrations of purified rPhl p 6, rPhl p 6 aa 1-57, rPhl p 6 aa 31-110, rPhl p 6 aa 1-33 or an anti-IgE mAb (E124.2.8 Dε2, Immunotech, Marseilles, France). Histamine released into the supernatant was measured by RIA (Immunotech)(Figure 6). Purified rPhl p 6 induced a specific and dose-dependent histamine release from basophils of a patient allergic to grass pollen, whereas rPhl p 6-derivatives aa 1-57 and aa 31-110 did not induce any histamine release up to a concentration of 1 µg/ml. Phl p 6 aa 1-33 induced a 50% release of histamine at a concentration of 1 µg/ml, which represents an approximately 1000 fold reduction of histamine release compared to complete rPhl p 6.

### DISCUSSION

Approximately 40% of allergic patients display immediate type symptoms after contact with grass pollen (3). We have isolated cDNAs coding for isoforms and fragments of a major timothy grass pollen allergen, designated Phl p 6. Phl p 6 represents a 11.8 kDa protein allergen which is recognized by IgE antibodies of 75% of grass pollen allergic patients. The prevalence of IgE recognition of rPhl p 6 is thus in accordance with that reported earlier for natural Phl p 6 and indicates that carbohydrate moieties do not play a relevant role in IgE recognition of Phl p 6 (28, 29). In agreement with peptide sequence data obtained for natural Phl p 6 we found that the deduced amino acid sequence of rPhl p 6 shows a high degree of sequence homology with the N-terminal portions of group 5 grass pollen allergens, a family of 25-35 kDa major grass pollen allergens (29, 14). Due to the presence of an N-terminal hydrophobic leader peptide, Phl p 6 represents an independent allergen, rather than a group 5 allergen fragment. In agreement with the proposal of other authors who analyzed a Phl p 6 encoding cDNA clone (15) we suggest that group 5 and group 6 allergens may have evolved from common ancestor genes similar as has been described for group 1 and group 2/3 grass pollen allergens (30). The assumption that Phl p 6 belongs to an independent group of grass pollen allergens is also supported by our finding that Phl p 6 shares few cross-reactive IgE epitopes with group 5 and no with other grass pollen allergens. The prediction of solvent accessibility indicated that many of the Phl p 6 N-terminal amino acids are solvent exposed while most of the C-terminal amino acid residues appeared to be buried. While no proof, this finding is in agreement with data obtained from the IgE epitope mapping experiments which indicate that the proteins N-terminus is critically involved in IgE recognition. It is however equally possible that the N-terminus itself represents a dominant IgE epitope or that deletion of the N-terminus affects conformational Phl p 6 IgE epitopes.

Expression of Phl p 6 in *E*. *coli* yielded large amounts of soluble and folded recombinant protein which contained almost exclusive alpha helical secondary structure. The alpha helical fold of Phl p 6 is a further confirmation that there are no common structural features which predispose a certain protein to behave as an allergen. While Phl p 6 is very likely an all alpha helical protein, Bet v 1, the major birch pollen allergen (31) and Bet v 2, birch profilin (32) have a mixed alpha beta fold. As revealed by CD spectroscopical analysis, rPhl p 6 shares with other immunologically unrelated pollen allergens (e. g., Bet v 1 (33), Bet v 2 (6, 32)) the remarkable intrinsic tendency to refold into a stable conformation after denaturation. Another feature that is shared by Phl p 6 and other important plant allergens is its high expression in pollen tissue. The fact that most of the plant allergens characterized so far are predominantly expressed in mature pollen may therefore be interpreted as a footprint of sensitization via the respiratory tract (34).

By immunogold electron microscopy, Phl p 6 was primarily localized on the P-particles of mature pollen. P-particles are small polysaccharide-containing bodies which represent up to 30% of the contents of the dormant pollen grain and, during pollen germination transfer material into the growing pollen-tube wall (35, 36). The occurence of Phl p 6 on the P-particles may be of clinical relevance as P-particles could act as small-sized (<2.5 micron) and therefore respirable allergen-carriers that bring Phl p 6 in immediate contact with the bronchial mucosa. A P-particle-linked intrusion of Phl p 6 into the deeper respiratory tract would thus explain the high prevalence (75%) of sensitization against this allergen although only a few grass species (*Phleum pratense, Poa pratensis*) contained rabbit anti-rPhl p 6-reactive moieties in the low (10-12 kDa) molecular weight range.

The *Escherichia coli* -expressed purified recombinant Phl p 6 allergen reacted with IgE antibodies of the majority of grass pollen allergic patients and induced basophil histamine release as well as immediate type skin reactions. It may therefore be used for *in vitro* as well as *in vivo* (skin test) diagnosis of grass pollen allergy. Our finding that deletion of the N-terminal portion of Phl p 6 dramatically reduced the allergens IgE binding capacity gave rise to the idea that it may be possible to construct Phl p 6 deletion variants which may be used for specific immunotherapy of grass pollen allergy with reduced anaphylactic side effects. A similar strategy was recently applied to disrupt the conformational IgE epitopes of the major birch pollen allergen Bet v 1 (23) but could not be predicted for Phl p 6 because the latter molecule contained continuous IgE epitopes. We produced N-terminally and C-terminally truncated versions of Phl p 6, of which the variant aa 1-57 and aa 31-110 showed almost completely abolished IgE binding capacity. We propose to use these two hypoallergenic Phl p 6 variants produced as recombinant molecules or by peptide chemistry as candidate vaccines against grass pollen allergy.

### TABLES AND FIGURES

**TABLE 1.** Immediate type skin reactivity to rPhl p 6. Four grass pollen allergic patients (HP, SF, CS, LW) and two non-allergic individuals (SV, SS) were skin tested with purified rPhl p 6, rPhl p 5, with natural timothy grass pollen extract, histamine and isotonic sodium chloride. Results are displayed as the mean diameters (mm) of the wheal reaction.
**TABLE II.** IgG-1-reactivity of mouse anti-rPhl p 6 or anti-rPhl p 6 aa 31-110 antisera to rPhl p 6.
**FIGURE 1****.** IgE reactivity of rPhl p 6 isoforms and fragments. Nitrocellulose filters containing proteins from recombinant λgt 11 phage expressing two Phl p 6 isoforms (c142, c223), Phl p 6 fragments (c121, c146, c171, c233) and for control purposes, λgt11 wild type phage (0) were probed with serum IgE from 9 grass pollen-allergic patients (1-9) and from one non-allergic individual (10).
**FIGURE 2****.** A, Purity of recombinant timothy grass pollen allergens. Coomassie brilliant blue-stained SDS-PAGE containing purified, recombinant timothy grass pollen allergens (Phl p 1, Phl p 2, Phl p 5, Phl p 6, timothy grass pollen profilin) and natural timothy grass pollen extract (Timothy). (M) Molecular weight marker. *B, C* Circular dichroism analysis. *B*, Far-UV circular dichroism spectra of rPhl p 6, expressed as mean residue ellipticity ([Θ]) (y-axis), were recorded in the wavelength range displayed on the x-axis at 20°C (continuous line), 85°C (dotted line) and at 20°C after cooling from 85°C (dashed line). C, Thermal denaturation and cooling of purified rPhl p 6 monitored at 220 nm (x-axis: temperature in °C; y-axis: apparent fraction of the folded protein).
**FIGURE 3****.** *A*, rPhl p 6 induces basophil histamine release. Granulocytes from a grass pollen allergic patient were incubated with various concentrations (x-axis) of purified, recombinant Phl p 6 (triangles), Phl p 5 (points) or a monoclonal anti-IgE antibody (squares). The percentage of histamine released into the supernatant is displayed on the y-axis. Results represent the means (+/- SD) of triplicate determinations. *B*, Induction of immediate type skin reactions with rPhl p 6 in sensitized allergic patients. Two grass pollen allergic patients (*a*) LW, (*b*) HP and a non-allergic individual (*c*) SV were pricked on their forearms with increasing concentrations of rPhl p 6 and rPhl p 5 as well as with histamine (Hist) and NaCI as indicated in (*d*). The wheal area was surrounded with a ball point pen.
**FIGURE 4****.** Tissue-specific expression of Phl p 6. A, Nitrocellulose-blotted grass pollen extracts from various monocots were probed with rabbit preimmune Ig (lanes 1) or rabbit anti-rPhl p 6 Ig (lanes 2). *B*, Comparable amounts of nitrocellulose blotted protein extracts from timothy grass pollen, leaves and roots were incubated with rabbit anti-profilin Ig (lanes 1), rabbit anti-Phl p 6 Ig (lanes 2) or rabbit preimmune Ig (lanes 3).
   C, *D,* Ultrastructural localization of Phl p 6. Ultrathin sections of timothy grass pollen were stained with rabbit anti-Phl p 6 Ig (*C*) and with rabbit preimmune Ig (*D*). Bound rabbit antibodies were detected with a gold-conjugated goat anti-rabbit Ig antiserum (gold particles = black dots). Arrows indicate Phl p 6 immunoreactivity on the P-particles. Abbreviations: E: exine; I: intine; P: P-particle. The bars represent 0.250 µm.
**FIGURE 5****.** Reduced IgE binding capacity of Phl p 6 deletion variants Equal amounts of recombinant Phl p 6 (*A*), Phl p 6 aa 1-57 (*B*) and Phl p 6 aa 31-110 (*C*) were tested for IgE-reactivity with sera from timothy grass pollen allergic patients (lane 1-13) and serum from a non-allergic control individual (lane 14). Lane 15 and lane 16 show the reactivity with a rabbit anti-Phl p 6 antiserum and a rabbit preimmunserum. Bound IgE antibodies were detected with ¹²⁵I-labeled anti-human IgE antibodies, bound rabbit antibodies with ¹²⁵I-labeled donkey anti-rabbit antibodies and visualized by autoradiography.
**FIGURE 6****.** Granulocytes from a patient allergic to grass pollen were incubated with various concentrations (1, 10⁻¹, 10⁻², 10⁻³, 10⁻⁵, and 10⁻⁷ µg/ml) of purified rPhl p 6 (points), rPhl p 6 aa 1-57 (up triangles), rPhl p 6 aa 31-110 (down triangles), rPhl p 6 aa 1-33 (rhombus) or an anti-IgE mAb (squares). Histamine released into the supernatant was measured by RIA and is displayed on the y-axis. Results represent the means of triplicate determinations.

### REFERENCES

1. Kay, A. B. 1997. Allergy and Allergic Diseases. Blackwell Science, Oxford, UK.
2. Segal, D. M., J. D. Taurog, and H. Metzger. 1977. Dimeric immunoglobulin E serves as a unit signal for mast cell degranulation. Proc. Natl. Acad. Sci. USA 41:457.
3. Freidhoff, L. R., E. Ehrlich-Kautzky, J. H. Grant, D. A. Meyers, and D. G. Marsh. 1986. A study of the human immune response to Lolium perenne (rye) pollen and its components, Lol p I and Lol p II (rye I and rye II). J. Allergy Clin. Immunol. 78:1190.
4. Niederberger, V., S. Laffer, R. Fröschl, D. Kraft, H. Rumpold, S. Kapiotis, R. Valenta, and S. Spitzauer. 1998. IgE antibodies to recombinant pollen allergens (Phl p 1, Phl p 2, Phl p 5, and Bet v 2) account for a high percentage of grass pollen-specific IgE. J. Allergy Clin. Immunol. 101:258.
5. Valenta, R., and D. Kraft. 1995. Recombinant allergens for diagnosis and therapy of allergic diseases. Curr. Opin. Immunol. 7: 751.
6. Valenta, R., M. Duchêne, K. Pettenburger, C. Sillaber, P. Valent, P. Bettelheim, M. Breitenbach, H. Rumpold, D. Kraft, and O. Scheiner. 1991. Identification of profilin as a novel pollen allergen; IgE autoreactivity in sensitized individuals. Science 253:557.
7. Seiberler, S., O. Scheiner, D. Kraft, D. Lonsdale, and R. Valenta. 1994. Characterization of a birch pollen allergen, Bet v 3, representing a novel class of Ca2+ binding proteins: specific expression in mature pollen and dependence of patients IgE binding on protein-bound Ca2+. EMBO J. 13:3481.
8. Shcherban, T. Y., J. Shi, D. M. Durachko, M. J. Guiltinan, S. J. McQueen-Mason, M. Shieh, and D. J. Cosgrove. 1995. Proc. Natl. Acad Sci. USA 92:9245.
9. Bufe, A., G. Schramm, M. B. Keown, M. Schlaak, and W. M. Becker. 1995. Major allergen Phl p 5b in timothy grass is a novel pollen RNase. FEBS Lett. 363:6.
10. Knox, R. B., C. Suphioglu, P. Taylor, R. Desai, H. C. Watson, J. L. Peng, and L. A. Bursill. 1997. Major grass pollen allergen Lol p 1 binds to diesel exhaust particles: implications for asthma and air pollution. Clin. Exp. Allergy 27:246.
11. Suphioglu, C., M. B. Singh, P. Taylor, R. Bellomo, P. Holmes, R. Puy, and R. B. Knox. 1992. Mechanism of grass pollen-induced asthma. Lancet 339:569.
12. Vallier, P., C. Dechamp, R. Valenta, O. Vial, and P. Deviller. 1992. Purification and characterization of an allergen from celery immunochemically related to an allergen present in several other plant species. Identification as a profilin. Clin. Exp. Allergy 22: 774.
13. Vrtala, S., M. Susani, W. R. Sperr, P. Valent, S. Laffer, C. Dolecek, D. Kraft, and R. Valenta. 1996. Immunologic characterization of purified recombinant timothy grass pollen (Phleum pratense) allergens (Phl p 1, Phl p 2, Phl p 5). J. Allergy Clin. Immunol. 97:781.
14. Vrtala, S., W. R. Sperr, I. Reimitzer, R. van Ree, S. Laffer, W.-D. Müller, P. Valent, K. Lechner, H. Rumpold, D. Kraft, O. Scheiner, and R. Valenta. 1993. cDNA cloning of a major allergen from timothy grass (Phleum pratense) pollen: characterization of the recombinant Phl p V allergen. J. Immunol. 151:4773.
15. Peterson, A., A. Bufe, G. Schramm, M. Schlaak, and W. M. Becker. 1995. Characterization of the allergen group VI in timothy grass pollen (Phl p 6). II. cDNA cloning of Phl p 6 and structural comparison to grass group V. Int. Arch. Allergy Immunol. 108:55.
16. Sambrook, J., E. F. Fritsch, and T. Maniatis. 1989. Molecular cloning: A Laboratory Manual. Cold Spring Harbor Laboratory Press, Cold Spring Harbor, NY.
17. Sanger, F., S. Nicklen, and A. R. Coulson. 1977. DNA sequencing with chain-terminating inhibitors. Proc. Natl. Acad. Sci. USA 74:5463.
18. Devereux, J., P. Haeberli, and O. Smithies. 1984. A comprehensive set of analysis programs for the VAX. Nucl. Acids Res. 12:387.
19. Thompson, J. D., D. G. Higgins, and T. J. Gibson. 1994. CLUSTAL W: improving the sensitivity of progressive multiple sequence alignment through sequence weighting, position-specific gap penalties and weight matrix choice. Nucl. Acids Res. 22:4673.
20. Rost, B., and C. Sander. 1993. Prediction of protein secondary structure at better than 70% accuracy. J. Mol. Biol. 232:584.
21. Ball, T., S. Vrtala, W. R. Sperr, P. Valent, M. Susani, D. Kraft, and R. Valenta. 1994. Isolation of an immunodominant IgE-hapten from an epitope expression cDNA library; dissection of the allergic effector reaction. J. Biol. Chem. 269:28323.
22. Valent, P., J. Besemer, M. Muhm, O. Maijdic, K. Lechner, and P. Bettelheim. 1989. Interleukin 3 activates human blood basophils via high-affinity binding sites. Proc. Natl. Acad. Sci. USA 86:5542.
23. Vrtala, S., K. Hirtenlehner, L. Vangelista, A. Pastore, H.-G. Eichler, W. R. Sperr, P. Valent, C. Ebner, D. Kraft, and R. Valenta. 1997. Conversion of the major birch pollen allergen, Bet v 1, into two nonanaphylactic T cell epitope-containing fragments; candidates for a novel form of specific immunotherapy. J Clin. Invest. 99:1673.
24. Laemmli, U. K. 1970. Cleavage of structural proteins during the assembly of the head of bacteriophage T4. Nature 227: 680.
25. Fling, S. P., and D. S. Gregerson. 1986. Peptide and protein molecular weight determination by electrophoresis using a high molarity tris buffer system without urea. Anal. Biochemi. 155:83.
26. Towbin, H., T. Staehelin, and J. Gordon. 1979. Electrophoretic transfers of proteins from polyacrylamide gels to nitrocellulose sheets: procedure and some applications. Proc. Natl. Acad. Sci. USA 76:4350.
27. Grote, M., C. Dolecek, R. vanRee, and R. Valenta. 1994. Immunogold electron microscopic localization of timothy grass (Phleum pratense) pollen major allergens Phl p 1 and Phl p 5 after anhydrous fixation in acrolein vapor. J. Histochem. Cytochem. 42:427.
28. Lowenstein, H. 1978. Isolation and partial characterization of three allergens of timothy pollen. Allergy 33:30.
29. Matthiesen, F., L. Friberg, M. Olsen, and H. Lowenstein. 1993. Purification and characterization of the Phleum pratense (timothy) pollen allergen Phl p 6. In: Molecular Biology and Immunology of Allergens. D. Kraft and A. Sehon eds. CRC Press, Boca Raton, pp. 189-191.
30. Dolecek, C., S. Vrtala, S. Laffer, P. Steinberger, D. Kraft, O. Scheiner, and R. Valenta. 1993. Molecular characterization of Phl p II, a major timothy grass (Phleum pratense) pollen allergen. FEBS Lett. 335: 299.
31. Gajhede, M., P. Osmark, F. M. Poulsen, H. Ipsen, J. N. Larsen, R. J. van Neerven, C. Schou, H. Lowenstein, and M. D. Spangfort. 1996. X-ray and NMR structure of Bet v 1, the origin of birch pollen allergy. Nature Struct. Biol. 3:1040.
32. Fedorov, A. A., T. Ball, R. Valenta, and S. C. Almo. 1997. Crystal structure and IgE epitope mapping of birch pollen profilin: The molecular basis for allergen cross-reactivity. Structure 5:33.
33. Laffer, S., L. Vangelista, P. Steinberger, D. Kraft, A. Pastore, and R. Valenta. 1996. Molecular characterization of Bip 1, a monoclonal antibody that modulates IgE binding to birch pollen allergen, Bet v 1. J. Immunol. 157:4943.
34. Valenta, R., and D. Kraft. 1996. Type I allergic reactions to plant-derived food: A consequence of primary sensitization to pollen allergens. J. Allergy Clin. Immunol. 97:893.
35. Heslop-Harrison, J., and Y. Heslop-Harrison. 1982. The growth of the grass pollen tube: 1. Characteristics of the polysaccharide particles ("P-particles") associated with apical growth. Protoplasma 112: 71.
36. Heslop-Harrison, J., Y. Heslop-Harrison, and J. S. Heslop-Harrison. 1997. Motility in ungerminated grass pollen: association of myosin with polysaccharide-containing wall-precursor bodies (P-particles). Sex. Plant Reprod. 10: 65.
37. Vrtala, S., Fischer, S., Grote, M., Vangelista, L., Pastore, A., Sperr, W., Valent, P., Reichelt, R., Kraft, D., Valenta, R. 1999. Molecular, immunological, and structural characterization of Phl p 6, a major allergen and P-particle-associated protein from Timothy grass (Phleum pratense) pollen. J. Immunol. 163:5489.

**Table I. Immediate type skin reactivity to rPhI p 6**

| Individual | Phl p 6 (10 µgml) | Phl p 6 (100 µg/ml) | Phl p 5 (10 µg/ml) | Phl p 5 (100 µg/ml) | Timothy grass | Histamine | NaCl |
|---|---|---|---|---|---|---|---|
| Patients allergic to grasspollen | | | | | | | |
| • HP | 5 | 16.5 | 5.5 | 13 | 16 | 5.5 | 0 |
| • SF | 0 | 13 | 2 | 11 | 8 | 7 | 0 |
| • CS | 0 | 12 | 5 | 8.5 | 12 | 9 | 0 |
| • LW | 2.5 | 10.5 | 5 | 13 | 9 | 5.5 | 0 |
| | | | | | | | |
| Non-allergic individuals | | | | | | | |
| • SV | 0 | 0 | | 0 | 0 | 6 | 0 |
| • SS | 0 | 0 | 0 | 0 | 0 | 7.5 | 0 |

**Table II.**

| **IgGl-reactivity of mouse anti-rPhl p 6 or anti-rPhl p 6 aa 31-110 antisera to rPhl p 6** | | | |
|---|---|---|---|
| Mouse anti-rPhl p 6 | Preimmuneserum | I. Immuneserum | II. Immuneserum |
| 1 | 0.060 | 0.445 | >2.5 |
| 2 | 0.061 | 1.528 | >2.5 |
| 3 | 0.065 | 0.253 | >2.5 |
| 4 | 0.061 | 0.508 | >2.5 |
| 5 | 0.062 | 0.864 | >2.5 |

| Mouse anti-rPhl p 6 aa31-110 | | | |
|---|---|---|---|
| 1 | 0.063 | 1.218 | >2.5 |
| 2 | 0.056 | >2.5 | >2.5 |
| 3 | 0.057 | 0.347 | >2.5 |
| 4 | 0.054 | >2.5 | >2.5 |
| 5 | 0.056 | 0.406 | >2.5 |

## Claims

1. A hypoallergenic immunogenic combination of molecules derived from the Phl p 6 allergen, comprising (i) a Phl p 6 molecule having an N-terminal deletion which makes the molecule at least substantially lack IgE binding capacity, and (ii) a Phl p 6 molecule having a C-terminal deletion which makes the molecule at least substantially lack IgE binding capacity, which two molecules together encompass the complete sequence of Phl p 6.

2. Use of an immunogenic molecule combination according to claim 1 in the preparation of a medicament for hyposensitization of a mammal suffering from IgE mediated allergy against the timothy grass pollen allergen Phl p 6.

3. Use of an immunogenic molecule combination according to claim 1 in the preparation of a medicament for specific immunotherapy for treatment of IgE-mediated grass pollen allergy.

4. The use of the immunogenic molecule combination according to claim 1, for the *in vitro* diagnosis of type I allergy in a mammalian individual.

5. The use of the immunogenic molecule combination according to claim 1, for the preparation of a medicament to be used in the hyposensitization of a mammalian individual suffering from a type I allergy, or for the preparation of a reagent to be used in diagnosis in *vivo* of type I allergy.

## Patentansprüche

1. Hypoallergene immunogene Kombination von von Phl p 6 abgeleitetem Allergen, umfassend (i) ein Phl p 6-Molekül, das eine N-terminale Deletion aufweist, die dem Molekül einen zumindest wesentlichen Mangel einer IgE-Bindungsfähigkeit verleiht, und (ii) ein Phl p 6-Molekül, das eine C-terminale Deletion aufweist, die dem Molekül zumindest einen wesentlichen Mangel an IgE-Bindungsfähigkeit verleiht, wobei beide Moleküle zusammen die vollständige Sequenz von Phl p 6 umfassen.

2. Verwendung einer immunogenen Molekül-Kombination nach Anspruch 1 zur Herstellung eines Arzneimittels zur Hypersensibilisierung eines Säugers, der von einer durch IgE-vermittelten Allergie gegen das Timotheusgras-Pollenallergen Phl p 6 leidet.

3. Verwendung einer immunogenen Molekül-Kombination nach Anspruch 1 zur Herstellung eines Arzneimittels für eine spezifische Immunotherapie zur Behandlung von durch IgE-vermittelter Graspollenallergie.

4. Verwendung der immunogenen Molekül-Kombination nach Anspruch 1 zur *in vitr*o-Diagnose von Typ I-Allergie in einem Säuger.

5. Verwendung der immunogenen Molekül-Kombination nach Anspruch 1 zur Herstellung eines Arzneimittels zur Verwendung in der Hyposensibilisierung eines Säugers, der an Typ I-Allergie leidet, oder zur Herstellung eines Reagens zur Verwendung in der *in vivo*-Diagnose von Typ I-Allergie.

## Revendications

1. Combinaison immunogène hypoallergénique de molécules dérivées de l'allergène Ph1 p6, comprenant (i) une molécule Ph1 p6 ayant une délétion du N- terminal qui fait en sorte que la molécule n'ait pas de capacité de liaison avec IgE et (ii) une molécule Ph1 p6 ayant une délétion du C-terminal qui fait en sorte que la molécule n'ait pas de capacité de liaison avec IgE, ces deux molécules comprenant ensemble la séquence complète de Ph1 p6.

2. Utilisation d'une combinaison de molécules immunogènes selon la revendication 1 dans la préparation d'un médicament pour l'hyposensibilisation d'un mammifère souffrant d'allergie, favorisée par IgE, vis- à- vis de l'allergène Ph1 p6 du pollen de la fléole des prés.

3. Utilisation d'une combinaison de molécules immunogènes selon la revendication 1 dans la préparation d'un médicament pour une immunothérapie spécifique au traitement de l'allergie au pollen de graminées favorisée par IgE.

4. Utilisation d'une combinaison de molécules immunogènes selon la revendication 1 pour le diagnostic in vitro d'une allergie de type I chez un mammifère.

5. Utilisation d'une combinaison de molécules immunogènes selon la revendication 1 pour la préparation d'un médicament utilisé pour l'hyposensibilisation d'un mammifère souffrant d'une allergie de type I ou pour la préparation d'un réactif utilisé dans un diagnostic in vivo d'une allergie de type I.
